(19) Europäisches Patentamt
European Patent Office
Office européen des brevets

(11) Veröffentlichungsnummer: **0 300 487 B1**

# (12) EUROPÄISCHE PATENTSCHRIFT

(45) Veröffentlichungstag der Patentschrift: **03.03.93**

(51) Int. Cl.[5]: **C12N 9/38**, C12N 15/56, G01N 33/535, G01N 33/58

(21) Anmeldenummer: **88111794.9**

(22) Anmeldetag: **21.07.88**

## (54) Enzymatisch inaktive, immunologisch aktive beta-Galactosidase-Muteine.

(30) Priorität: **24.07.87 DE 3724625**

(43) Veröffentlichungstag der Anmeldung:
**25.01.89 Patentblatt 89/04**

(45) Bekanntmachung des Hinweises auf die Patenterteilung:
**03.03.93 Patentblatt 93/09**

(84) Benannte Vertragsstaaten:
**AT BE CH DE ES FR GB GR IT LI LU NL SE**

(56) Entgegenhaltungen:

**ANALYTICAL BIOCHEMISTRY, Band 146, 1985; R.ARMENTA et al., Seiten 211-219**

**BIOCHEMICAL AND BIOPHYSICAL RESEARCH COMMUNICATIONS, Band 152, Nr. 3, Mai 1988; M.RING et al., Seiten 1050-1055**

**BIOCHEMICAL AND BIOPHYSICAL RESEARCH COMMUNICATIONS, Band 153, Nr. 1, Mai 1988; D.E.BADER et al., Seiten 301-306**

(73) Patentinhaber: **BOEHRINGER MANNHEIM GMBH
Patentabteilung, Abt. E Sandhofer Strasse 112-132 Postfach 31 01 20
W-6800 Mannheim 31 Waldhof(DE)**

(72) Erfinder: **Mattes, Ralf, Prof.Dr.
Friedrich Zundel-Str. 14
W-7000 Stuttgart 75(DE)**
Erfinder: **Lenz, Helmut, Dr.
v. Kühlmann-Str. 14
W-8132 Tutzing(DE)**
Erfinder: **Stock, Werner, Dr.
Grawolfstr. 2
W-8032 Gräfelfing(DE)**

(74) Vertreter: **Huber, Bernhard, Dipl.-Chem. et al
Patentanwälte H. Weickmann, Dr. K. Fincke F.A. Weickmann, B. Huber Dr. H. Liska, Dr. J. Prechtel Kopernikusstrasse 9 Postfach 86 08 20
W-8000 München 86 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die Erfindung betrifft enzymatisch inaktive, immunologisch aktive β-Galactosidasemuteine, ihre Herstellung und ihre Verwendung für Enzymimmunassays (EIA) mit β-Galactosidase als Markierungsenzym zur Beseitigung von Serumstöreinflüssen und zur Senkung von unspezifischen Blankwerten.

Es wurde beobachtet, daß bei Enzymimmunassays Humanseren mit geringem tatsächlichem Analytgehalt manchmal hohe Signale ergeben. Diese Störungen lassen sich auf Serumfaktoren zurückführen, die mit Antianalyt-β-Galactosidase-Konjugat Bindungen eingehen, die nicht der eigentlich beabsichtigten Anti-Analyt-Bindungsfunktion des Konjugats entsprechen. Solchen Seren wurden folglich falsche Analytwerte zugeordnet.

Enzymimmunassays können in verschiedener Weise durchgeführt werden. Sie sind sowohl als Sandwichverfahren als auch in homogener löslicher Phase möglich.

Bei Verwendung des Sandwich-Verfahrens ist die Bestimmung der Menge an gewünschtem Serumprotein einfach: Der in fester Phase vorliegende Antigen-Antikörper-Enzymkomplex wird von der überstehenden Lösung abgetrennt und darauffolgend die Enzymaktivität gemessen.

Schwieriger ist jedoch die Messung der Menge an fraglichem Serumprotein bei homogenen Enzymimmunoassays unter Verwendung von β-Galactosidase. Hierbei stehen mehrere Möglichkeiten zur Auswahl: z. B. ein colorimetrischer Enzyminhibierungs-Immunoassay (Gibbons et al in Immobilized Enzyms and Cells, Methods in Enzymology, K. Moosbach, ed., Academic Press, New York), ein turbidometrischer Enzymaktivierungs-Immunoassay (Gibbons et al, Clin. Chem., 27:16002 (1981), und der empfindlichere fluorimetrische Enzyminhibitions-Immunoassay (Armenta et al, Anal. Biochem., (1985)).

Bei allen Methoden können jedoch Störfaktoren im Serum, d. h. Proteine, die an das β-Galactosidasemolekül selbst oder an den damit verbundenen Anti-Analyt-Antikörper oder auch deren Verbindungsstellen binden, sowie auch darin enthaltene Inhibitoren der β-Galactosidase-Aktivität die quantitative Bestimmung erheblich beeinträchtigen. Solche Inhibitoren können Anti-β-Galactosidase-Antikörper sein, die die Enzymaktivität durch Blockieren des aktiven Zentrums für das makromolekulare Substrat des Enzyms inhibieren.

Um eine solche Beeinträchtigung der Genauigkeit des Enzymimmunoassays zu verhindern, wurden mehrere Methoden entwickelt. So wurde der Antikörper-Enzymkomplex vor Anti-Enzym-Antikörper durch Ankopplung von mehreren Albuminmolekülen an das Enzym "geschützt". Interessanterweise kann dies ohne eine Beeinträchtigung der Enzymaktivität durchgeführt werden. Jedoch ergibt sich dadurch möglicherweise eine sterische Inhibition nicht nur der Bindung eines Anti-Enzym-Antikörpers, sondern auch der gewünschten Bindung an das zu bestimmende Protein. Außerdem ist es bekannt, inaktive β-Galactosidase-Derivate,die den nativen antigenischen Charakter des Enzyms aufweisen, im Überschuß einzusetzen, um so die Bindung des Anti-Enzym-Antikörper an die aktive β-Galactosidase zu verhindern. Auch mit dieser Methode konnte der Test jedoch nicht mit der angestrebten Genauigkeit durchgeführt werden.

Der Erfindung liegt daher die Aufgabe zugrunde, die vorstehend geschilderten Schwierigkeiten zu beseitigen und eine Methode zur Eliminierung von Störfaktoren aus dem Probenserum bereitzustellen. Die Erfindung löst diese Aufgabe durch das Bereitstellen eines enzymatisch inaktiven, immunologisch aktiven β-Galactosidasemutein-Proteins, bei dem im Bereich zwischen den Aminosäuren 430 und 550 mindestens eine Aminosäure der natürlichen Sequenz gegen eine andere Aminosäure ausgetauscht ist und die enzymatische Aktivität nicht mehr als 1 %, bezogen auf das native Enzym, beträgt.

In bevorzugten Ausführungsformen findet dieser Aminosäureaustausch an den Aminosäuren Glu 461 und/oder der Aminosäure Tyr 503 statt. Beispielsweise kann die Aminosäure Glu 461 gegen eine der Aminosäuren Ser, Ala, Asp oder Tyr und/oder die Aminosäure Tyr 503 gegen eine der Aminosäuren Phe, Glu oder Ser ausgetauscht werden. Jedoch ist auch der Austausch gegen jeweils eine andere Aminosäure denkbar. Durch diesen Aminosäureaustausch im angegebenen Bereich wird die Enzymaktivität auf 1 %, bevorzugt 1 o/oo der Enzymaktivität der nativen β-Galactosidase reduziert, die imunologische Aktivität der β-Galactosidase jedoch nicht beeinträchtigt.

Die Herstellung dieser β-Galactosidase-Muteine kann auf verschiedene Arten durchgeführt werden. Erfindungsgemäß wird die Herstellung über site-directed mutagenesis eines für β-Galactosidase kodierenden Plasmids auf DNA-Ebene an den entsprechenden Stellen und darauffolgender Expression in geeigneten Wirten durchgeführt. Die sitedirected mutagenesis kann günstigerweise nach der Heteroduplextechnik (Morinega et al, Biotechnology, Juli 1984, 636 (1984)) bewirkt werden. Hierzu wird ein für β-Galactosidase kodierendes Plasmid mit Restriktionsendonukleasen auf zwei verschiedene Arten gespalten: zum einen wird durch Spaltung mit den Enzymen A und B der für β-Galactosidase kodierende Bereich aus dem Plasmid herausgeschnitten, es bleibt ein lineares Plasmid übrig, welches keine β-Galactosidase-Sequenzen enthält; zum anderen wird dasselbe Plasmid mit einem Restriktionsenzym C geschnitten, welches außerhalb der für β-Galactosidase kodierenden Region schneidet, wodurch das Plasmid linearisiert wird. Sodann wird durch

Denaturierung durch Erhitzen ein Aufschmelzen der doppelsträngigen DNA-Struktur bewirkt, was zur Bildung von Einzelstrang-DNA-Ketten führt. Dieser Lösung von einzelsträngigen DNA-Fragmenten wird ein phosphoryliertes Oligonukleotid zugegeben, welches einen kleinen Teil der β-Galactosidasesequenz darstellt, jedoch an der Stelle des gewünschten Aminosäureaustausches eine Mutation von einer Base des für die Aminosäure kodierenden Triplets enthält. Nach Renaturierung der das Oligonukleotid enthaltenden Einzelstrang-DNA- Mischung durch schrittweises Abkühlen besteht die Möglichkeit der Bildung von vier verschiedenen doppelsträngigen DNA-Molekülen I, a, b und II. Die doppelsträngigen DNA-Moleküle I und II stellen nur Rückbildungen der Ausgangsmoleküle durch Hybridisierung dar. Bei den Molekülen a und b sind nicht die beiden Teilstränge der Ausgangsmoleküle wieder miteinander vereint worden, sondern jeweils ein Einzelstrang der Ausgangsmoleküle I und II. Es entstehen daher doppelsträngige DNA-Moleküle, die im für β-Galactosidase kodierenden Bereich einzelsträngig sind. Daher kann sich an das Molekül a, welches den Komplementärstrang für die DNA-Sequenz des Oligonukleotids enthält, das Oligonukleotid durch Hybridisierung anheften, es entsteht dabei jedoch im Bereich des Basenaustauschs ein sogenanntes "missmatch". Durch Zugabe von DNA-Polymerase I und den 4 Desoxynukleotiden sowie von $T_4$-DNA-Ligase werden die restlichen einzelsträngigen Regionen aufgefüllt und die doppelsträngige DNA geschlossen. Besonders günstig ist es, den Basenaustausch so durchzuführen, daß in dem entsprechenden Plasmid das Schnittmuster bei Verdau mit einem Restriktionsenzym verändert wird, wodurch nach Transformation die Selektionierung der das mutierte Plasmid enthaltenden Kolonien erleichtert wird. Dies kann entweder durch Zerstören oder Hinzufügen einer Restriktionsschnittstelle für ein Restriktionsenzym durch Auswahl einer geeigneten Base geschehen. Die Bewertung der Kolonien kann jedoch auch noch über andere bekannte Techniken wie Hybridisationsanalyse (Kolonien/Plasmide), Färbung der Kolonien mit X-gal, SDS-Gelelektrophorese ganzer Zellextrakte, Immunassays mit Schaf-anti-β-gal-IgG-POD-Konjugat, native Gelelektrophorese oder Enzymaktivitätstests mit verschiedenen anderen Farbsubstraten durchgeführt werden. Die das mutierte Plasmid enthaltenden (positiven) Kolonien werden weiter vermehrt, wobei der zelleigene Reperaturmechanismus das "missmatch" auf zwei Arten reparieren kann, nämlich so, daß einmal das Mutantenplasmid und zum anderen wieder Wildtypplasmid entsteht. Die neuerlich erhaltenen Kolonien werden nach den obengenannten Techniken beurteilt und das Mutantenplasmid zur Expression der β-Galactosidase-Muteine in geeigneten Wirtszellen verwendet. Man hat festgestellt, daß bei allen in den Beispielen durchgeführten Aminosäureaustauschen die Aggregation der β-Galactosidase-Mutein-Monomere zu Tetrameren nicht beeinträchtigt war.

Die β-Galactosidase-Muteine werden zusammen mit dem Fab-Teil eines Immunoglobulins, welches jedoch nicht für den Analyt bindungsaktiv ist, eingesetzt. Dadurch können im Serum eventuell vorhandene Störfaktoren gegen bindungsaktive Fab-Teile des Antianalyt-Immunoglobulins ebenso, wie Störfaktoren, die gegen das β-Galactosidaseprotein gerichtet sind, abgefangen werden. Bei Einsatz des chemisch verbundenen Konjugats, wobei die beiden Bestandteile β-Galactosidase-Mutein und Fab-Teil des nicht Analytbindenden Immunoglobulins durch das gleiche Verfahren, das für aktive Konjugate verwendet wird, nämlich z. B. Bindung über N-Maleinimidohexanoyl-O-Succinimid, verbunden sind, anstelle des separaten Einsatzes von Mutein und Fab, ist als weiterer Vorteil zu nennen, daß die Sicherheit des Tests weiter gesteigert wird, da hierbei auch solche Störfaktoren kompensiert werden, welche gegen Fab bzw. gegen die Bindungsstelle zwischen Mutein und Fab gerichtet sind, d. h. gegen den Brückenbildner selbst oder die Verbindungsstelle zwischen Brückenbildner und den beiden miteinander verknüpften Substanzen.

Ein weiterer Gegenstand der Erfindung ist ein Reagenz für Enzymimmunoassays (EIA) mit β-Galactosidase als Markierungsenzym zur Beseitigung von Serumstöreinflüssen und zur Senkung von unspezifischen Blankwerten, welches dadurch gekennzeichnet ist, daß es ein enzymatisch inaktives, immunologisch aktives β-Galactosidasemutein enthält und wenn es zusammen mit dem Fab-Teil eines Analyt-nicht-bindenden Immunoglobulins, möglichst in chemisch gebundener Form, eingesetzt wird, alle Eigenschaften des Anti-Analyt-Konjugats, auf welche sich unspezifische Bindungen von Serumstörfaktoren richten können, exakt simuliert, ohne jedoch enzymatisch oder antianalytaktiv zu sein. Dadurch werden Serumstörfaktoren abgefangen und können die nachfolgende Bestimmung mit enzymatisch aktiver β-Galactosidase nicht mehr beeinträchtigen.

Die folgenden Beispiele erläutern die Erfindung näher in Verbindung mit der Zeichnung:
In der Zeichnung stellen dar:

- Fig. 1 ein Anionenaustauscher-Chromatogramm gemäß Beispiel 2.3.1
- Fig. 2 ein Gelfiltrationschromatogramm gemäß Beispiel 2.3.2
- Fig. 3 ein Polyacrylamidgelelektropherogramm gemäß Beispiel 2.3.3
- Fig. 4 a und b je ein Vernetzungprofil von
  - a) Gal Mutein Phe 503

b) nativer β-Galactosidase mit Maus-Fab

Fig. 5 eine graphische Darstellung der Immunreaktivität von Gal Muteinen und nativer β-Gal

Fig. 6 eine schematische Darstellung des im Beispiel 4 und 5 verwendeten Zentrifugal-Rotoreinsatzelementes

**Beispiel 1**

Die molekularbiologischen Standardmethoden wie z. B. Transformation, Restriktion und Plasmidisolierung werden nach Maniatis, T., Fritsch, E.F., and Sambrook, J. (1982) "Molecular Cloning", Cold Spring Harbor Laboratory, New York, 11724, durchgeführt.

Konstruktion von β-Gal-Mutein-Genen

1a) Herstellung des Ausgangsplasmids

Aus den Genen des E.coli-Phagen λ plac 5-1 (DSM 4176P), beschrieben in C. Pourcel et al., Molec. gen. Genet. 170 (1979), 161-169, wird durch Spaltung mit dem Restriktionsenzym Bst EII ein ca. 5,2 kb-Fragment erhalten und präparativ durch Gelelektrophorese gewonnen. Das Plasmid pACYC 177 (DSM 3693P) wird durch Spaltung mit dem Enzym Pst I linearisiert. Die Enden des lacZ tragenden ca. 5,2 kb großen BstEII-Fragmentes aus λ plac 5-1 werden unter Einsatz des Enzyms Nuclease S1 glatt verdaut und mit Hilfe des Enzyms terminale Transferase und dCTP am 3′ Ende um eine beliebige Anzahl von d-Cytidin-Resten verlängert (Verfahrensbeschreibung siehe Maniatis et al., Seiten 217 bis 246). Das mit Pst I linearisierte pACYC 177 Molekül wird unter Einsatz des Enzyms terminale Transferase und GTP am 3′ Ende um eine beliebige Anzahl von d-Guanidin-Resten verlängert. Die Mischung beider so verlängerter Fragmente wird vereinigt und so durch Transformation in E.coli-Zellen des Stammes BMTU 2489 (DSM 4178) eingeführt. Die E.coli-Zellen werden auf Nährmedium, das 25 µg/ml Kanamycin und 40µg/ml 5-Brom-4-chlor-3-indolyl-β-D-galaktosid (X-Gal) enthält, selektioniert. Aus den Zellen, die blau und sensitiv gegen 50 µg/ml Ampicillin sind, wird das Plasmid isoliert, bei dem beide Enden des ursprünglichen Bst E II-Fragments um 15 G/C-Paare verlängert sind. Dieses Plasmid wird als Ausgangsplasmid verwendet. Dieses unterscheidet sich von λ plac 5-1 und pACYC 177 dadurch, daß es durch Spaltung mit Pst I zwei Fragmente erhalten läßt, von denen das größere das lacZ-Gen-Fragment aus λ plac 5-1 enthält.

Zellen von E.coli ,die das Ausgangsplasmid enthalten, produzieren konstitutiv β-Gal.

Ebenso als Ausgangsplasmid geeignet sind Plasmide, bei denen beide Enden des Bst E II-Fragments um 5 bis 30 G/C-Paare verlängert sind.

1a′) Herstellung eines alternativen Ausgangsplasmids

Aus den Genen des E.coli-Phagen λplac 5-1 (DSM 4176P) wird durch Spaltung mit dem Restriktionsenzym Bst EII ein ca. 5 kb-Fragment erhalten und präparativ durch Gelelektrophorese gewonnen. Das Plasmid pACYC 177 (DSM 3693P) wird durch Spaltung mit dem Enzym Pst I linearisiert. Die Enden des lacZ tragenden ca. 5 kb großen BstEII-Fragmentes aus λplac 5-1 werden unter Einsatz des Enzyms Nuclease S1 glatt verdaut. Die beiden Fragmente werden ligiert und in E. coli transformiert.

Die E. coli-Zellen werden auf Nährmedium, das 25 µg/ml Kanamycin und 40 µg/ml 5-Brom-4-chlor-3-indolyl-β-D-galaktosid (X-Gal) enthält, selektioniert. Zellen, die blau sind und sensitiv gegen 50 µg/ml Ampicillin sind, werden ausgewählt. Diese Zellen enthalten jeweils eines von zwei verschiedenen Plasmiden. Diese Plasmide unterscheiden sich durch die Orientierung des Bst E II-Fragments und ergeben demzufolge mit BamHI und EcoRI verschiedene Spaltmuster. Man wählt als alternatives Ausgangsplasmid dasjenige aus, welches nach Verdau mit BamHI und EcoRI eine Länge von ca. 9,1 kb hat.

1b) Herstellung des Mutagenese-Mixes und Mutagenese-Prozedur

Das nach 1a oder 1a′ hergestellte Ausgangsplasmid wird mit den Enzymen Bcl I und Sac I behandelt und das größere Fragment (ca. 8,2 bp lang) präparativ durch Gelelektrophorese gewonnen. In einem weiteren Ansatz wird das Ausgangsplasmid durch Spaltung mit Hind III linearisiert. Beide Plasmidfragmente werden gemischt (je 0,15 pmol) und durch Erhitzen auf 95 °C für 2 min. denaturiert. Dabei werden vorher zusätzlich 75 pmol eines kinasierten Oligo-Nukleotides zugefügt. Die Sequenz des Oligo-Nukleotides bestimmt die zu erhaltene Mutein-Species und wird unten näher erläutert (vgl. Formeln I bis III). Durch Behandlung dieses Gemisches bei 60 °C für 30′ wird die Ausbildung von Heteroduplex-Molekülen erreicht.

Die Mutagenese Prozedur wird anschließend bei 12°C unter Zusatz von folgenden Reagentien auf die angegebene Endkonzentration durchgeführt: Desoxy-Nukleotid-Triphosphate 0,25 mM, ATP 1 mM, NaCl 0,1 M, Tris-HCl pH 7,5 6,5 mM; $MgCl_2$ 8 mM, β-Mercaptoäthanol 1 mM, Klenow-Fragment 0,125 U/µl, T4-Ligase 0,0625 U/µl.

Die Inkubationszeit beträgt 4 Stunden. Anschließend wird ein Aliquot des Ansatzes, enthaltend 0,0075 pmol Vektor, zur Transformation in E.coli-Zellen BMTU 2489 (DSM 4178) verwendet. Die transformierten Kolonien werden auf Nährmedium mit 25 µg/ml Kanamycin und 40 µg/ml X-Gal selektioniert. Die gewünschten Mutanten sind β-Gal negativ (weiß) und werden mit einer Rate von 8 % gefunden. Die gereinigten Kolonien werden zur Plasmid-Präparation verwendet, die so erhaltenen Plasmide wie oben beschrieben, retransformiert. Die so erhaltenen weißen zweiten Transformanten-Zellen werden zur Plasmidpräparation verwendet und die Plasmid-DNA mit dem verwendeten radioaktiv markierten Oligo-Nukleotid hybridisiert. Plasmide, die durch Waschen der Hybridisationsfilter bei 50°C noch positive radioaktive Resultate zeigen, sind Plasmide, die die gewünschte Mutation enthalten. Aus Zellen, die diese Plasmide enthalten, werden Rohextrakte präpariert. Diese enthalten geringe Werte von β-Gal-Aktivität (<0,5 % der Zellen mit Ausgangsplasmid). Auf SDS-Gelen werden diese Rohextrakte durch Elektrophorese aufgetrennt und mit Coomassie-Farbstoff die Proteinbanden angefärbt.Die Rohextrakte aus solchen Plasmid-tragenden Zellen, die das gewünschte β-Gal-Mutanten-Gen enthalten, weisen eine prominente β-Gal-Bande auf, die identisch mit der aus Rohextrakten des Ausgangsplasmids im Gel wandert und gleiche Größe aufweist.

1c) Herstellung des Muteins β-Gal $Tyr_{503}$ → Glu

Verwendet wird ein Oligo-Nukleotid der Sequenz

5′ TGC CCG ATG <u>GAA</u> GCG CGC GTG 3′,   (I)

das an der Aminosäurecodonposition 503 GAA statt TAC im Wildtyp lacZ-Gen enthält. Dadurch wird an dieser Position ein Glu-Rest statt Tyr codiert. Plasmide, die diese gewünschte Mutation tragen, besitzen eine Rsa I-Schnittstelle weniger als das Ausgangsplasmid.

1d) Herstellung des Muteins β-Gal $Glu_{461}$ → Ala und $Tyr_{503}$ → Phe

Verwendet werden die beiden Oligo-Nukleotide der Sequenz

5′ TG GGG AAT <u>GCA</u> TCA GGC CA 3′,   (II)

das an der Aminosäurecodonposition 461 GCA statt GAA im Wildtyp lacZ-Gen enthält und

5′ TGC CCG ATG <u>TTT</u> GCG CGC GTG 3′,   (III)

das an der Aminosäurecodonposition 503 TTT statt TAC im Wildtyp lacZ-Gen enthält.

Plasmide, die diese gewünschte Mutation tragen, besitzen eine Hinf I und eine Rsa I-Schnittstelle weniger als das Ausgangsplasmid.

**Beispiel 2**

Vergleich von nativer β-Galactosidase und Gal Muteinen

2.1 Reinigung aus E.coli Biomasse

Biomasse von E.coli mit genetischer Information für native β-Gal bzw. Biomasse der transformierten E.coli-Stämme enthaltend ein Plasmid, das nach 1c oder 1d erhalten wurde, wird in geeignetem Nährmedium in 10 l-Fermentoren hergestellt. Die Isolierung der β-Gal wird nach dem Verfahren von G. R. Craven, E. Steers Jr., C.B. Anfinsen, J. Biol. Chem. 240 (1965) 2468-2477 ausgeführt. Es enthält die Schritte Ammoniumsulfatfällung, Gelfiltration und Anionenaustauscher-Chromatographie. Die Gal Muteine verhalten sich bei Aufreinigung nach dem selben Verfahren auf allen Reinigungsstufen nicht unterscheidbar von nativer β-Gal Wegen fehlender Enzymaktivität wird die Anreicherung der Muteine durch hochauflösende Gelfiltration an einer TSK G4000 Säule (siehe 2.3.1) kontrolliert.

Das hohe Molekulargewicht von Gal Proteinen bedingt auf dem Hintergrund der übrigen E.coli Proteine einen sehr charakteristischen Proteinpeak, der über alle Reinigungsstufen quantitativ ausgewertet werden kann.

2.2 Enzymatische Aktivität

2.2.1 Testvorschrift zur Bestimmung der Enzymaktivität von ß-Galactosidase

$$\text{2-Nitrophenyl-ß-D-galactopyranosid} + H_2O \xrightarrow{\text{ß-Galactosidase}} \text{D-Galactose} + \text{2-Nitrophenol}$$

Gemessen wird das gebildete 2-Nitrophenol bei Hg 405 nm.

LÖSUNGEN:

1. Kaliumphosphat-Puffer (0,05 mol/l; pH 7,8):
   a) 0,68 g $KH_2PO_4$ in bidest. Wasser lösen und auf 100 ml auffüllen
   b) 1,14 g $K_2HPO_4 \cdot 3 H_2O$ in bidest. Wasser lösen und auf 100 ml auffüllen
   Lösung b) mit Lösung a) auf pH-Wert von 7,8 einstellen.
2. Magnesiumchlorid-Lösung (10 mmol/l):
   203,3 mg $MgCl_2 \cdot 6 H_2O$ (M 5833) in 100 ml bidest. Wasser lösen.
3. Nitrophenyl-ß-D-galactopyranosid-Lösung:
   5,9 mg 2-NP-ß-D-galactosid in 1 ml Puffer (1) lösen (die Lösung darf nur schwach gelb gefärbt sein).
4. Mercaptoäthanol-Lösung:
   69,8 ml 2-Mercaptoäthanol mit bidest. Wasser auf 100 ml auffüllen.

Probelösung:
Stammlösungen von nativer ß-Galactosidase bzw. ß-Gal-Mutein werden in Puffer 1 verdünnt.

Ausführung

Meßstrahlung: Hg 405 nm; Schichtdicke: 1 cm;
Testvolumen: 2,98 ml; Temperatur: 37 ° C.

| In Plastik-Kuevetten pipettieren: | Leerwert | Probe |
|---|---|---|
| Puffer (1)<br>$MgCl_2$ (2)<br>2-NP-galactosid (3)<br>Mercaptoäthanol (4) | 2,20 ml<br>0,300 ml<br>0,400 ml<br>0,030 ml | 2,20 ml<br>0,300 ml<br>0,400 ml<br>0,030 ml |
| mischen, temperieren und vorgegebene Temperatur in der Kuevette überprüfen. Reaktion starten mit | | |
| Puffer (1)<br>Probe | 0,050 ml<br>- | -<br>0,050 ml |
| mischen, Extinktionsänderung verfolgen und ΔE/min aus dem linearen Bereich berechnen. | | |

Berechnung

$$\text{Aktivität} = \frac{2{,}98}{0{,}05} \cdot \Delta E/\text{min} \, [\text{U/ml Probe-Lösung}]$$

$$\varepsilon_{405} = 3{,}5 \, [\text{mmol}^{-1} \cdot \text{l} \cdot \text{cm}^{-1}]$$

2.2.2 Ergebnisse der Aktivitätsbestimmungen

Native β-Gal 600-650 U/mg Protein
Gal Mutein(Glu503) < 0,2 U/mg Protein
Gal Mutein(Ala461/Phe503) < 0,2 U/mg Protein

2.3 Analyse durch hochauflösende analytische Methoden.

2.3.1 Anionenaustauscher-Chromatographie

Trennsäule MonoQ HR5/5 (Pharmacia)
Puffer A 20 mM Tris/HCl pH 7,6
Puffer B 20 mM Tris/HCl, 1 M NaCl pH 7,6
Flußrate 1 ml/min.
Papiervorschub 0,5 cm/min
Gradientenform siehe Figur 1
UV-Monitor 280 nm
Raumtemperatur
Ergebnis: Native β-Gal und typische Gal Muteine wie Gal Mutein(Glu503) oder Gal Mutein (Ala461/Phe503) ergeben ununterscheidbar das Chromatogramm der Figur 1.

2.3.2 Gelfiltration

Trennsäule TSK G4000 SW, 7,5 x 300 mm (LKB)
Puffer 0,1 M Kaliumphosphat/0,1 % Natriumazid, pH 6,8
Flußrate 1 ml/min
Papiervorschub 0,5 cm/min
UV-Monitor 280 nm
Raumtemperatur
Ergebnis: Native β-Gal und typische Gal Muteine wie Gal Mutein(Glu503) oder Gal Mutein(Ala461/Phe503) ergeben ununterscheidbar das Chromatogramm der Figur 2. Durch Eichung der Trennsäule mit Eichproteinen bekannten Molekulargewichts läßt sich der nativen β-Gal und den Gal Muteinen ein Molekulargewicht von ca. 520000 zuordnen.

2.3.3 Polyacrylamid Gelelektrophorese

Elektrophoreseapparatur: Phast-System (Pharmacia)
Gradientengel 8-25 % (Pharmacia)
Pufferbedingungen für native Elektrophorese
Durchführung nach Handbuch Phast-System von Pharmacia Färbung mit Coomassie-Farbstoff.
Ergebnis: Die nach 2.1 gereinigten Präparate von nativer β-Gal und von Gal Muteinen ergeben nicht unterscheidbares Bandenmuster wie ersichtlich aus Figur 3.

2.4 Charakterisierung von Gal Muteinen durch Verhalten bei Kupplung mit Maus-Fab

Gereinigtes Präparat von Gal Mutein wurde mit Maus-Fab gekuppelt wie beschrieben in Beispiel 3. Vom Kupplungsansatz wurde vor der Gelchromatographie an Sepharose 6B eine Probe (50 µl) durch hochauflösende Gelchromatographie an TSK G4000 analysiert. Das sich ergebende Vernetzungsprofil, siehe Figur 4a,

kennzeichnet die Molekulargewichtsverteilung des Konjugats Maus-Fab-Gal Mutein. Figur 4b zeigt ein entsprechendes Profil für einen Konjugatansatz von Maus-Fab gekoppelt mit nativer β-Gal.
Ergebnis: Gal Mutein(Glu503) verhält sich bei Kupplung mit Maus-Fab genauso wie native β-Gal; d.h. die SH-Gruppen des Gal Muteins sind für die Verbrückung mit dem Maleinimid Kupplungsreagenz genauso zugänglich wie jene von nativer β-Gal. Analoge Befunde wurden für das Gal Mutein(Phe503) erhalten.

Der Unterschied der Profile 4a und 4b ist nicht signifikant, da für verschiedene native β-Gal Chargen und verschiedene Kupplungsansätze ähnliche kleine Unterschiede unvermeidlich sind.

2.5 Prüfung der Immunreaktivität gegenüber Schaf-Anti β-Gal Antikörper

2.5.1 Testvorschrift

Testprinzip: An den Kunststoff von Vertiefungen einer Mikrotiterplatte wird ein polyklonaler Schaf-anti-β-Gal-IgG-Antikörper adsorbiert. Proben einer Verdünnungsreihe von β-Gal bzw. Gal Mutein werden in solche beschichtete Vertiefungen eingefüllt. Immunreaktives Protein bindet sich proportional der Konzentration der Verdünnung an die jeweilige Kunststoffwand. Nach Waschen der Vertiefungen wird das jeweils gebundene immunreaktive Protein mit einer Lösung Peroxidase-markiertem Schaf-anti-β-Gal-Fab versetzt. Proportional zur bereits wandgebundenen Menge immunreaktivem Gal-Protein bindet nun Peroxidase-Konjugat an die Festphase. Nach erneuter Waschung wird die jeweils wandgebundene POD-Aktivität durch Substratzugabe (ABTS$^{R}$) entwickelt und mit einem Photometer für Mikrotiterplatten quantitativ gemessen.

Testdurchführung:

1. Schritt: In jede Vertiefung einer Mikrotiterplatte 0,2 ml einer Lösung von 10 µg Schaf-anti-β-Gal IgG pro 1 ml 40 mM Kaliumphosphatpuffer pH 7,4 pipettieren. 60 min. bei Raumtemperatur inkubieren.
2. Schritt: Jede Vertiefung mit einer Kanüle aussaugen und mit 0,3 ml einer Lösung von 10 mg Rinderserumalbumin in Puffer A (50 mM Kaliumphosphatpuffer/l00 mM NaCl, pH 7,5) füllen. 60 min bei Raumtemperatur inkubieren.
3. Schritt: Vertiefungen leersaugen und je 2 Vertiefungen füllen mit 0,2 ml von Verdünnungen enthaltend 0, 5, 10, 20, 30, 40 ng β-Gal bzw. Gal Mutein/1 ml Puffer A enthaltend 0,1 % Rinderserumalbumin. 60 min bei Raumtemperatur inkubieren.
4. Schritt: Alle Vertiefungen aussaugen, mit je 0,3 ml Puffer A füllen und erneut aussaugen. Dann je Vertiefung 0,2 ml einer Lösung von Peroxidase-Schaf-anti β-Gal Fab in Puffer A/0,1 % Rinderserumalbumin pipettieren. (Peroxidasegehalt 100 mU/ml). 60 min. bei Raumtemperatur inkubieren.
5. Schritt: Alle Vertiefungen aussaugen, 2 x waschen mit Puffer A. Dann jeweils 0,2 ml Substratlösung mit 100 mg ABTS$^{R}$ (Fa. Boehringer Mannheim GmbH) in 0,1 M Kaliumphosphat/Zitratpuffer, pH 4,4 einfüllen. 60 min bei Raumtemperatur inkubieren.
6. Schritt: Extinktion der Farblösung in den Vertiefungen mit einem Photometer für Mikrotiterplatten bei 405 nm messen.

Auswertung:
Zusammengehörige Extinktionsdoppelwerte für jede Proteinverdünnung mitteln und auf der Ordinate eines Diagramms eintragen über der zugehörigen, auf der Abzisse angetragenen Proteinkonzentration. Punkte durch Ausgleichskurve verbinden. Typische Graphen, die für β-Gal bzw. Gal Muteine mit dieser Testführung erhalten werden, zeigt Fig. 5.

2.5.2 Ergebnis

Figur 5 zeigt graphisch die Abhängigkeit der Extinktionen im Enzymimmun-Test von der Proteinmenge an β-Gal bzw. Gal Mutein. Die Kurven für die untersuchten Gal Muteine sind im Rahmen der Meßfehlergrenze identisch mit jener von nativer β-Gal. D.h. alle Muteine werden von einem Antikörper, der durch Immunisierung von Schafen mit nativer β-Gal gewonnen ist, exakt genauso erkannt, wie das Immunogen native β-Gal. Anders ausgedrückt, die Immundeterminanten auf der Oberfläche der Muteine sind nicht unterscheidbar von denen nativer β-Gal.

**Beispiel 3**

Herstellung von Maus-Fab-β-Galactosidase-Konjugat

Die kovalente Kopplung von β-Galactosidase an Fab-Fragmente folgt den Vorgaben von T. Kitagawa in "Enzyme Immunoassay", E. Ishikawa, T. Kawai, K. Miyai Editors, Igaku-Shoin, Tokyo/New York 1981, Seite 81-89:
10 mg Fab-Protein werden in 2 ml 0,05 M Phosphatpuffer pH 7,0 gelöst und mit 75 µl einer Lösung N-(m-Maleinimidobenzoyloxy)succinimid (5 mg/ml) in Tetrahydrofuran versetzt. Das Gemisch wird 30 Minuten bei Raumtemperatur inkubiert und gelegentlich umgeschüttelt. Durch Passage des Reaktionsansatzes über eine Gelchromatographiesäule mit 15 ml Sephadex G25-Füllung (Pharmacia) wird das umgesetzte Fab vom Reagenzüberschuß abgetrennt. Betriebspuffer ist 10 mM Phosphatpuffer pH 6,2.

Zu dem maleinimidohexanoylierten Fab (8 mg Fab-Protein) werden 20 mg β-Galactosidase (E.coli) in 2 ml 0,075 M Phosphatpuffer pH 7,0 gegeben und 2 Stunden bei Raumtemperatur inkubiert. Das Reaktionsprodukt wird durch Gelchromatographie an Sepharose 6B (Pharmacia) gereinigt. Säulendimension 2 x 50 cm; Chromatographiepuffer 0,02 M Phosphat/1 mM $MgCl_2$/0,1 % Natriumazid, 0,1 % NaCl). Die aktiven Konjugatfraktionen eluieren von der Säule bei Molekulargewichten von 590.000 - ca. 2.500.000. Die für den Test geeignetsten Fraktionen werden daraus durch probeweise Verwendung in Testansätzen ermittelt. Die günstigsten Fraktionen werden zu einem Pool vereinigt.

**Beispiel 4**

Bestimmung von Thyreotropin (TSH) in Humanseren bei Anwesenheit von Störfaktoren gegen das Markerenzym β-Galactosidase

Die Bestimmung wird mit Trockenchemiereagensträger in einem Einschrittest nach dem Doppel-Antikörper Festphase-Sandwich-Prinzip (DASP) in Rotoreinsatzelementen mit einem Zentrifugalanalysator mit der in der DE-AS 34 25 008.5 beschriebenen Analyseapparatur durchgeführt.

1. Herstellung der Reagenslösungen

1.1 Puffer I

50 mmol/l Kalium-Phosphat-Puffer, pH 6,0, hergestellt durch Mischung von 50 mmol/l $K_2HPO_4$-Lösung und 50 mmol/l $KH_2PO_4$-Lösung bis zum Erreichen des pH-Wertes 6,0.

1.2 Puffer II

Puffer II wird wie Puffer I hergestellt mit dem Unterschied, daß der pH-Wert auf 7,5 eingestellt wird und daß der Puffer zusätzlich 10 g/l Rinderserumalbumin und 150 mmol/l NaCl enthält.

1.3 Rezeptor $R_1$-Lösung, bindefähig mit TSH

Als Rezeptor $R_1$ wird ein monoklonaler Maus-anti-TSH-Antikörper eingesetzt. Die diesen Antikörper enthaltende Ascitesflüssigkeit wird ad 1,8 mol/l mit Ammoniumsulfat versetzt. Das Präzipitat wird in einem Puffer aus 15 mmol/l Natriumphosphat, pH 7,0 und 50 mmol/l Natriumchlorid aufgenommen. Die so erhaltene, mit TSH-bindefähige Lösung wird einer Passage über DEAE-Cellulose unterworfen. Das so erhaltene, TSH-bindefähigen Antikörper enthaltende Eluat wird mit Puffer II auf eine Protein-Konzentration von 1 µg/ml verdünnt.

1.4 Enzymmarkierte Rezeptor $R_3$-Lösung

Als Rezeptor $R_3$ wird ebenfalls ein monoklonaler Maus-Anti-TSH-Antikörper eingesetzt, der jedoch eine andere antigene Determinante als Rezeptor $R_1$ erkennt. Die diesen Antikörper enthaltende Ascitesflüssigkeit wird, wie unter 1.3 angegeben, aufgereinigt. Der vollständige Antikörper wird in bekannter Weise nach der Methode von R.R. Porter, Biochem. J. 73, (1959), S. 119, zum Fab-Fragment gespalten. Die erhaltenen Fab-Fragmente werden gemäß Beispiel 3 mit β-Galactosidase gekoppelt. Die Rezeptor $R_3$-β-gal-Konjugat-Lösung wird in Puffer II auf eine Konzentration von 500 mU/ml (gemessen mit o-Nitrophenyl-β-Galactosid

bei 37° C) verdünnt.

1.5 Inaktive Enzymlösung; β-gal-Mutein (Glu 503)

Das durch gezielte Veränderung des nativen Markerenzyms β-Galctosidase (in Rezeptor $R_3$) durch Punktmutation in Kodon 503 erzeugte β-gal- Mutein (Glu 503), welches enzymatisch inaktiv ist, aber in seiner Oberflächenstruktur unverändert ist, und somit die antigenen Determinanten wie das native Enzym besitzt, wird in Puffer II auf eine Konzentration von 1 mg/ml verdünnt.

1.6 Rezeptor $R_2$-Lösung

Schaf-anti-Maus-Fc γ-Antiserum wird ad 1,8 mol/l mit Ammoniumsulfat versetzt. Das Präzipitat wird in einem Puffer aus 15 mmol/l Natriumphosphat, pH 7,0 und 50 mmol/l Natriumchlorid aufgenommen. Die so erhaltene Lösung wird einer Passage über DEAE-Zellulose unterworfen. Das den spezifischen Antikörper enthaltende Eluat wird in Puffer I auf eine Proteinkonzentration von 50 µg/ml verdünnt.

1.7 Substratlösung

| | |
|---|---|
| Chlorphenolrot-β-galactosid (hergestellt nach DE 33 45 748) | 5 mmol/l (3,05 g/l) |
| HEPES | 70 mmol/l (16,7 g/l) |
| NaCl | 154 mmol/l (9 g/l) |
| Rinderserumalbumin | 0,3 % (3 g/l) |
| Tween 20 | 0,2 % (2 g/l) |
| pH (mit NaOH) | 7,25 |

2. Herstellung von Reagensträgern

2.1 Reagensträger 1 (ohne Mutein)

40 µl einer Lösung, die pro Liter 100 mmol Natriumphosphat pH 7,3 (37° C), 2 mmol Magnesiumchlorid, 9 g Natriumchlorid, 5 g Rinderserumalbumin, 5 mg Anti-TSH monoklonale Antikörper aus Maus (Rezeptor $R_1$), 1000 U Anti-TSH-Antikörper-(Maus)-Fab-Fragment-β-Galactosidase-Konjugat (Rezeptor-$R_3$-Konjugat-Lösung; Aktivität bestimmt mit ortho-Nitrophenyl-β-D-galactosid bei 37° C) enthält, wird auf ein Vlies aufgetropft, das aus kommerziellem Polyesterpapier besteht. Anschließend wird bei Raumtemperatur getrocknet. Diese Vliese werden bis zu ihrer Verwendung bei 4° C und einer relativen Luftfeuchtigkeit von 20 % aufbewahrt.

2.2 Reagensträger 1′ (Zusatz von Mutein)

Die Herstellung erfolgt wie für Reagensträger 1 bis auf den Unterschied, daß zusätzlich pro Liter Tränklösung 25 mg β-gal-Mutein (Glu 503) enthalten sind.

2.3 Reagensträger 2

Auf ein Zellulosevlies werden nach dem Bromcyan-Aktivierungsverfahren (DE-OS 1768512) Schaf-Antikörper gegen den Fc γ-Teil von Maus-Antikörpern (Rezeptor-$R_2$-Lösung) fixiert, wobei pro Gramm Fasermaterial 10 µg Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagensträger 1.

3. Durchführung der Bestimmung

Die Bestimmung mit Hilfe dieser beiden Reagensträger 1 und 2, bzw. 1′ und 2, erfolgt mit der in der DE-AS 3 425 008.5 beschriebenen Vorrichtung zur Durchführung analytischer Bestimmungen.

Diese lehrt ein in Fig. 6 dargestelltes Rotoreinsatzelement für Zentrifugalanalysenautomaten, bestehend aus einem Formkörper, der eine Probenauftragskammer, die mit einer Mehrzahl von Reagensfeldern in Verbindung steht, die jeweils ein mit einem bestimmten Reagensimprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist, und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist.

Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (VK1) zu einer zweiten Ventilkammer (VK2) und von dieser über die Kammer (d) und über eine Auffangkammer (AK) zur Meßkammer (K). Zur Aufnahme einer weiteren Flüssigkeit ist eine als Pumpenkammer ausgebildete Substraktkammer (PK) vorgesehen, welche über eine aus Dosierkammer (DK) und Kapillare (Kap) bestehende Dosiereinrichtung und eine Überlaufkammer (ÜK) mit der zweiten Ventilkammer (VK2) verbunden ist.

Zur Bestimmung der Extinktion a (Meßsignal inklusive Störsignal) werden Reagensträger 1 und Reagensträger 2 benutzt, zur Bestimmung der Extinktion b (spezifisches Meßsignal ohne Storsignal) werden Reagensträger 1′ und 2 benutzt.

Reagensträger 1 bzw. 1′ wird auf Feld c des Rotoreinsatzelementes plaziert und Reagensträger 2 auf Feld d. Dabei werden 40 µl konzentrierter Probe durch eine Öffnung am oberen Rand direkt auf das Feld a pipettiert. 270 µl Substratlösung werden in Kammer PK piepttiert. Durch ein geeignetes Zentrifugationsprogramm, bei dem hohe Drehzahlen mit Stillstand abwechseln, werden dann Probe und Substratlösung in Richtung Trennmatrix und Küvette gefördert.

Dabei werden im Verlauf des Programms die Rezeptoren $R_1$ und $R_3$ ohne bzw. mit Mutein durch die Probenflüssigkeit vom Feld c eluiert und die homogene Mischung anschließend zur Reaktion gebracht. Auf Feld d werden die gebildeten Komplexe über $R_1$ an den Rezeptor $R_2$ gebunden. Der Transfer der Probe von Feld c nach d erfolgt innerhalb sehr kurzer Zeit.

Die Substratlösung wird durch die Dosierkammer DK in Portionen geteilt, von denen die ersten zum Auswaschen von überschüssigem, nichtkomplexiertem Konjugat dienen.

Störfaktoren, die $R_3$ vernetzen könnten, werden bei Einsatz von 20fachem Überschuß an β-gal-Mutein gegenüber dem nativen Markerenzym neutralisiert und ebenfalls ausgewaschen (Einsatz von Reagensträger R1′).

Die über Komplexbildung auf d gebundene β-Galactosidase-Aktivität ist proportional zur in der Probe enthaltenen TSH-Menge bzw. zum Probenleerwert. Diese Aktivität wird mit einer weiteren Substratportion bestimmt, wobei das Substrat in einer 5minütigen Reaktion zu farbigen Produkten umgesetzt wird. Die gebildete Farbe und die weitere Farbentwicklung/min in der flüssigen Phase werden in der Küvette bei 576 nm gemessen.

Unter diesen Bedingungen wurden folgende Resultate erhalten:

| Probe | a | | b | |
|---|---|---|---|---|
| | Ext. [mE] | Konz. [µU/ml] | Ext. [mE] | Konz. [µU/ml] |
| TSH Kalibrator 0 µU/ml[c] | 451 | 0 | 457 | 0 |
| TSH-Kalibrator 19,6 µU/ml[c] | 3331 | 19,6 | 3311 | 19,5 |
| Humanserum 1 | 669 | 1,5 | 725 | 1,8 |
| Humanserum 2 mit anti Gal-Störfaktoren | 2573 | 14,4 | 649 | 1,3 |

Alle Messungen wurden bei λ = 576 nm bei einer Schichtdicke von 0,3 cm durchgeführt und auf Schichtdicke d = 1 cm umgerechnet.

a) TSH-Bestimmung bei Verwendung von Reagensträger 1

b) TSH-Bestimmung bei Verwendung von Reagensträger 1′ mit β-gal-Mutein zur Neutralisation von Galactosidase-spezifischen Störfaktoren

c) TSH-Standards kalibriert am 2. IRP 80/588.

**Beispiel 5**

Bestimmung von Thyreotropin (TSH) in Humanseren Senkung unspezifischer Blankwerte

Die Bestimmung wird analog Beispiel 4 durchgeführt. Alle Reagenzlösungen sind identisch bis auf die im Beispiel 4 unter 1.5 beschriebene Lösung. Hier wird in diesem fall eine mit inaktivem β-Galactosidase Mutein markierte, analytunspezifische Fab-Konjugatlösung verwendet.

Herstellung:

Das durch gezielte Veränderung des nativen Markerenzyms β-Galactosidase (in Rezeptor $R_3$) durch Punktmutation in Kodon 503 erzeugte β-Gal-Mutein (Glu503), welches enzymatisch inaktiv ist, aber in seiner Oberflächenstruktur unverändert ist, und somit die antigenen Determinanten wie das native Enzym besitzt, wird wie im Beispiel 4 unter 1.4 beschrieben mit dem Fab-Teil eines Immunglobulins, welches jedoch nicht für den Analyt bindungsaktiv ist gekoppelt. Das so erhaltene Mutein-Fab-Konjugat wird in Puffer II auf eine Konzentration von 1 mg/ml verdünnt.

Auch die Herstellung der Reagensträger und die Durchführung der Bestimmung erfolgt analog Beispiel 4. Serumfaktoren, die unspezifische Blankwerte verursachen, werden bei Einsatz von überschüssigem (immunologisch wie enzymatisch inaktivem Mutein-Fab-Konjugat) neutralisiert und ebenfalls ausgewaschen (Einsatz von Reagensträger R1′).

Die über Komplexbildung auf d gebundene β-Galactosidase-Aktivität ist proportional zur in der Probe enthaltenen TSH-Menge bzw. zum Probenleerwert.

Diese Aktivität wird mit einer weiteren Substratportion bestimmt, wobei das Substrat in einer 5minütigen Reaktion zu farbigen Produkten umgesetzt wird. Die gebildete Farbe und die weitere Farbentwicklung/min in der flüssigen Phase werden in der Küvette bei 576 nm gemessen.

Unter diesen Bedingungen wurden folgende Resultate erhalten:

| Probe | a | b | Δ % |
|---|---|---|---|
| TSH freies [c] Humanserum | 485±32 [mE] | 339±11 [mE] | - 30 % |
| Humanserum [c] 19,6 µU/ml TSH-Gehalt | 3983±166 [mE] | 3733±1072 [mE] | - |
| Eichkurvensteilheit | 178 [mE/µU] | 173 [mE/µU] | - 3 % |

Alle Messungen wurden bei λ = 576 nm bei einer Schichtdicke von 0,3 cm durchgeführt und auf Schichtdicke d = 1 cm umgerechnet.

a) TSH-Bestimmung bei Verwendung von Reagensträger 1

b) TSH-Bestimmung bei Verwendung von Reagensträger 1′ mit Mutein-Fab-Konjugat zur Reduzierung von unspezifischen Blankwerten.

c) TSH Standards kalibriert am 2. IRP 80/588.

**Patentansprüche**
**Patentansprüche für folgende Vertragsstaaten : AT, BE, CH, DE, FR, GB, IT, LI, LU, NL, SE**

1. Enzymatisch inaktives, immunologisch aktives β-Galactosidase-Mutein, **dadurch gekennzeichnet,** daß im Bereich zwischen den Aminosäuren 430 und 550 mindestens eine Aminosäure der natürlichen Sequenz gegen eine andere Aminosäure ausgetauscht ist und die enzymatische Aktivität nicht mehr als 1 %, bezogen auf das native Enzym, beträgt.

2. β-Galactosidase-Mutein nach Anspruch 1, **dadurch gekennzeichnet,** daß die Aminosäure Glu 461 und/oder die Aminosäure Tyr 503 gegen eine andere Aminosäure ausgetauscht sind.

3. β-Galactosidase-Mutein nach Anspruch 2, **dadurch gekennzeichnet,** daß die Aminosäure Glu 461 gegen eine der Aminosäuren Ser, Ala, Asp, Tyr und/oder die Aminosäure Tyr 503 gegen eine der Aminosäuren Phe, Glu oder Ser ausgetauscht sind.

4. Verfahren zur gentechnologischen Herstellung eines enzymatisch inaktiven, immunologisch aktiven β-Galactosidase-Muteinproteins nach Anspruch 1, **dadurch gekennzeichnet,** daß in einem für β-Galactosidase kodierenden Plasmid die DNA-Sequenz durch site-directed mutagenesis derart verändert wird, daß nach Expression im Bereich zwischen den Aminosäuren 430 und 550 des Proteins mindestens eine Aminosäure gegen eine andere ausgetauscht ist.

5. Verfahren nach Anspruch 4, **dadurch gekennzeichnet,** daß der Aminosäureaustausch an der Aminosäure Glu 461 und/oder der Aminosäure Tyr 503 durchgeführt wird.

6. Verfahren nach Anspruch 5, **dadurch gekennzeichnet,** daß die Aminosäure Glu 461 gegen eine der Aminosäuren Ser, Ala, Asp, Tyr und/oder die Aminosäure Tyr 503 gegen eine der Aminosäuren Phe, Glu oder Ser ausgetauscht werden.

7. Verwendung eines enzymatisch inaktiven, immunologisch aktiven β-Galactosidase-Muteins gemäß einem der Ansprüche 1 bis 3, bei der immunologischen Bestimmung von Serumproteinen nach dem Prinzip des Enzym-Immunoassays (EIA) unter Verwendung des Enzyms β-Galactosidase als Markierungsenzym zum Abfangen von Anti-β-Galactosidase-Antikörpern oder anderer auf die β-GAL gerichteter Bindeproteine, die das Testergebnis verfälschen können.

8. Verwendung nach Anspruch 7, **dadurch gekennzeichnet,** daß das enzymatisch inaktive β-Galactosidasemutein zusammen mit einem Fab-Teil von einem Immunglobulin, das nicht bindungsaktiv für den Analyt ist, eingesetzt wird.

9. Verwendung nach Anspruch 8, **dadurch gekennzeichnet,** daß das enzymatisch inaktive β-Galactosidasemutein mit einem Fab- Teil eines Immunoglobulins, welches nicht bindungsaktiv für den Analyt ist, chemisch gebunden vorliegt.

10. Verwendung nach Anspruch 8, **dadurch gekennzeichnet,** daß ein Gemisch aus dem Fab-Teil eines Immunoglobulins, das nicht bindungsaktiv für den Analyt ist, und dem enzymatisch inaktiven β-Galactosidasemutein eingesetzt wird.

11. Reagenz für Enzymimmunoassays (EIA) mit β-Galactosidase als Markierungsenzym zur Beseitigung von Serumstöreinflüssen und zur Senkung von unspezifischen Blankwerten, **dadurch gekennzeichnet,** daß es ein enzymatisch inaktives, immunologisch aktives β-Galactosidasemutein nach Anspruch 1, 2 oder 3 enthält.

12. Reagenz nach Anspruch 11, **dadurch gekennzeichnet,** daß es das enzymatisch inaktive, immunologisch aktive β-Galactosidasemutein zusammen mit dem Fab-Teil eines Immunoglobulins, das für den Analyt nicht bindungsaktiv ist, enthält.

13. Reagenz nach Anspruch 12, **dadurch gekennzeichnet,** daß β-Galactosidasemutein und Fab-Teil eines Immunoglobulins, das nicht bindungsaktiv für den Analyt ist, als Gemisch vorliegen.

14. Reagenz nach Anspruch 12, **dadurch gekennzeichnet,** daß es das β-Galactosidasemutein und den Fab-Teil eines Immunoglobulins, das nicht bindungsaktiv für den Analyt ist, in chemisch gebundener Form enthält.

**Patentansprüche für folgende Vertragsstaaten : ES, GR**

1. Verfahren zur gentechnologischen Herstellung eines enzymatisch inaktiven, immunologisch aktiven β-Galactosidase-Muteinproteins **dadurch gekennzeichnet,** daß in einem für β-Galactosidase kodierenden Plasmid die DNA-Sequenz durch site-directed mutagenesis derart verändert wird, daß nach Expression im Bereich zwischen den Aminosäuren 430 und 550 des Proteins mindestens eine Aminosäure gegen eine andere ausgetauscht ist und die enzymatische Aktivität nicht mehr als 1 %, bezogen auf das native Enzym, beträgt.

2. Verfahren nach Anspruch 1, **dadurch gekennzeichnet,** daß der Aminosäureaustausch an der Aminosäure Glu 461 und/oder der Aminosäure Tyr 503 durchgeführt wird.

3. Verfahren nach Anspruch 2, **dadurch gekennzeichnet,** daß die Aminosäure Glu 461 gegen eine der Aminosäuren Ser, Ala, Asp, Tyr und/oder die Aminosäure Tyr 503 gegen eine der Aminosäuren Phe, Glu oder Ser ausgetauscht werden.

4. Verwendung eines enzymatisch inaktiven, immunologisch aktiven β-Galactosidase-Muteinswelches gemäß einem der Ansprüche 1 bis 3 hergestellt ist, bei der immunologischen Bestimmung von Serumproteinen nach dem Prinzip des Enzym-Immunoassays (EIA) unter Verwendung des Enzyms β-Galactosidase als Markierungsenzym zum Abfangen von Anti-β-Galactosidase-Antikörpern oder anderer auf die β-GAL gerichteter Bindeproteine, die das Testergebnis verfälschen können.

5. Verwendung nach Anspruch 4, **dadurch gekennzeichnet,** daß das enzymatisch inaktive β-Galactosidasemutein zusammen mit einem Fab-Teil von einem Immunglobulin, das nicht bindungsaktiv für den Analyt ist, eingesetzt wird.

6. Verwendung nach Anspruch 5, **dadurch gekennzeichnet,** daß das enzymatisch inaktive β-Galactosidasemutein mit einem Fab- Teil eines Immunoglobulins, welches nicht bindungsaktiv für den Analyt ist, chemisch gebunden vorliegt.

7. Verwendung nach Anspruch 5, **dadurch gekennzeichnet,** daß ein Gemisch aus dem Fab-Teil eines Immunoglobulins, das nicht bindungsaktiv für den Analyt ist, und dem enzymatisch inaktiven β-Galactosidasemutein eingesetzt wird.

8. Reagenz für Enzymimmunoassays (EIA) mit β-Galactosidase als Markierungsenzym zur Beseitigung von Serumstöreinflüssen und zur Senkung von unspezifischen Blankwerten, **dadurch gekennzeichnet,** daß es ein enzymatisch inaktives, immunologisch aktives β-Galactosidasemutein welches nach Anspruch 1, 2 oder 3 hergestellt ist, enthält.

9. Reagenz nach Anspruch 8 , **dadurch gekennzeichnet,** daß es das enzymatisch inaktive, immunologisch aktive β-Galactosidasemutein zusammen mit dem Fab-Teil eines Immunoglobulins, das für den Analyt nicht bindungsaktiv ist, enthält.

10. Reagenz nach Anspruch 9 **dadurch gekennzeichnet,** daß β-Galactosidasemutein und Fab-Teil eines Immunoglobulins, das nicht bindungsaktiv für den Analyt ist, als Gemisch vorliegen.

11. Reagenz nach Anspruch 9 **dadurch gekennzeichnet,** daß es das β-Galactosidasemutein und den Fab-Teil eines Immunoglobulins, das nicht bindungsaktiv für den Analyt ist, in chemisch gebundener Form enthält.

**Claims**

**Claims for the following Contracting States : AT, BE, CH, DE, FR, GB, IT, LI, LU, NL, SE**

1. Enzymatically-inactive, immunologically-active β-galactosidase mutein, characterised in that, in the region between the amino acids 430 and 550, at least one amino acid of the natural sequence is exchanged for another amino acid and the enzymatic activity does not amount to more than 1%, referred to the native enzyme.

2. β-Galactosidase mutein according to claim 1, characterised in that the amino acid Glu 461 and/or the amino acid Tyr 503 are exchanged for another amino acid.

3. β-Galactosidase mutein according to claim 2, characterised in that the amino acid Glu 461 is exchanged for one of the amino acids Ser, Ala, Asp, Tyr and/or the amino acid Tyr 503 is exchanged for one of the amino acids Phe, Glu or Ser.

4. Process for the gene-technological production of an enzymatically-inactive, immunologically-active β-galactosidase mutein protein according to claim 1, characterised in that, in a plasmid coding for β-galactosidase, the DNA sequence is changed by site-directed mutegenesis in such a manner that, after expression, in the region between the amino acids 430 and 550 of the protein, at least one amino acid is exchanged for another one.

5. Process according to claim 4, characterised in that the amino acid exchange is carried out on the amino acid Glu 461 and/or on the amino acid Tyr 503.

6. Process according to claim 5, characterised in that the amino acid Glu 461 is exchanged for one of the amino acids Ser, Ala, Asp, Tyr and/or the amino acid Tyr 503 is exchanged for one of the amino acids Phe, Glu or Ser.

7. Use of an enzymatically-inactive, immunologically-active $\beta$-galactosidase mutein according to one of claims 1 to 3 in the immunological determination of serum proteins according to the principle of the enzyme immunoassay (EIA) with the use of the enzyme $\beta$-galactosidase as labelling enzyme for the capture of anti-$\beta$-galactosidase antibodies or of other binding proteins directed to the $\beta$-GAL which can falsify the test result.

8. Use according to claim 7, characterised in that the enzymatically-inactive $\beta$-galactosidase mutein is used together with a Fab part of an immunoglobulin which is not binding-active for the analyte.

9. Use according to claim 8, characterised in that the enzymatically-inactive $\beta$-galactosidase mutein is present chemically bound with a Fab part of an immunoglobulin which is not binding-active for the analyte.

10. Use according to claim 8, characterised in that there is used a mixture of the Fab part of an immunoglobulin which is not binding-active for the analyte and the enzymatically-inactive $\beta$-galactosidase mutein.

11. Reagent for enzyme immunoassays (EIA) with $\beta$-galactosidase as labelling enzyme for the removal of serum disturbing influences and for the lowering of non-specific blank values, characterised in that it contains an enzymatically-inactive, immunologically-active $\beta$-galactosidase mutein according to claim 1, 2 or 3.

12. Reagent according to claim 11, characterised in that it contains the enzymatically-inactive, immunologically-active $\beta$-galactosidase mutein together with the Fab part of an immunoglobulin which is not binding-active for the analyte.

13. Reagent according to claim 12, characterised in that the $\beta$-galactosidase mutein and Fab part of an immunoglobulin which is not binding-active for the analyte are present as mixture.

14. Reagant according to claim 12, characterised in that it contains the $\beta$-galactosidase mutein and the Fab part of an immunoglobulin which is not binding-active for the analyte in chemically bound form.

**Claims for the following Contracting States : ES, GR**

1. Process for the gene-technological production of an enzymatically-inactive, immunologically-active $\beta$-galactosidase mutein protein, characterised in that, in a plasmid coding for $\beta$-galactosidase, the DNA sequence is changed by site-directed mutagenesis in such a manner that, after expression, in the region between the amino acids 430 and 550 of the protein, at least one amino acid is exchanged for another one and the enzymatic activity amounts to not more than 1%, referred to the native enzyme.

2. Process according to claim 1, characterised in that the amino acid exchange is carried out on the amino acid Glu 461 and/or on the amino acid Tyr 503.

3. Process according to claim 2, characterised in that the amino acid Glu 461 is exchanged for one of the amino acids Ser, Ala, Asp, Tyr and/or the amino acid Tyr 503 is exchanged for one of the amino acids Phe, Glu or Ser.

4. Use of an enzymatically-inactive, immunologically-active $\beta$-galactosidase mutein which is produced according to one of claims 1 to 3 in the immunological determination of serum proteins according to the principle of the enzyme immunoassay (EIA) with the use of the enzyme $\beta$-galactosidase as labelling enzyme for the capture of anti-$\beta$-galactosidase antibodies or of other binding proteins directed to the $\beta$-

GAL which can falsify the test result.

5. Use according to claim 4, characterised in that the enzymatically-inactive β-galactosidase mutein is used together with a Fab part of an immunoglobulin which is not binding-active for the analyte.

6. Use according to claim 5, characterised in that the enzymatically-inactive β-galactosidase mutein is present chemically bound with a Fab part of an immunoglobulin which is not binding-active for the analyte.

7. Use according to claim 5, characterised in that there is used a mixture of the Fab part of an immunoglobulin which is not binding-active for the analyte and the enzymatically-inactive β-galactosidase mutein.

8. Reagent for enzyme immunoassays (EIA) with β-galactosidase as labelling enzyme for the removal of serum disturbing influences and for the lowering non-specific blank values, characterised in that it contains an enzymatically-inactive, immunologically-active β-galactosidase mutein according to claim 1, 2 or 3.

9. Reagent according to claim 8, characterised in that it contains the enzymatically-inactive, immunologically-active β-galactosidase mutein together with the Fab part of an immunoglobulin which is not binding-active for the analyte.

10. Reagent according to claim 9, characterised in that the β-galactosidase mutein and Fab part of an immunoglobulin which is not binding-active for the analyte are present as mixture.

11. Reagent according to claim 9, characterised in that it contains the β-galactosidase mutein and the Fab part of an immunoglobulin which is not binding-active for the analyte in chemically bound form.

**Revendications**

**Revendications pour les Etats contractants suivants : AT, BE, CH, DE, FR, GB, IT, LI, LU, NL, SE**

1. Mutéine de β-galactosidase enzymatiquement inactive et immunologiquement active, caractérisée en ce que, dans le domaine compris entre les acides aminés 430 et 550, au moins un acide aminé de la séquence naturelle est remplacé par un autre acide aminé et l'activité enzymatique ne dépasse pas 1% par rapport à l'enzyme native.

2. Mutéine de β-galactosidase selon la revendication 1, caractérisée en ce que l'acide aminé Glu 461 et/ou l'acide aminé Tyr 503 sont remplacés par un autre acide aminé.

3. Mutéine de β-galactosidase selon la revendication 2, caractérisée en ce que l'acide aminé Glu 461 est remplacé par l'un des acides aminés Ser, Ala, Asp, Tyr et/ou l'acide aminé Tyr 503 est remplacé par l'un des acides aminés Phe, Glu ou Ser.

4. Procédé de préparation par génie génétique d'une protéine mutéine de β-galactosidase enzymatiquement inactive et immunologiquement active selon la revendication 1, caractérisé en ce que la séquence d'ADN d'un plasmide codant pour la β-galactosidase est modifiée par mutagenèse dirigée de telle manière qu'après l'expression au moins un acide aminé est remplacé par un autre dans le domaine compris entre les acides aminés 430 et 550 de la protéine.

5. Procédé selon la revendication 4, caractérisé en ce que l'échange d'acides aminés est opéré au niveau de l'acide aminé Glu 461 et/ou de l'acide aminé Tyr 503.

6. Procédé selon la revendication 5, caractérisé en ce que l'acide aminé Glu 461 est remplacé par l'un des acides aminés Ser, Ala, Asp, Tyr et/ou l'acide aminé Tyr 503 est remplacé par l'un des acides aminés Phe, Glu ou Ser.

7. Utilisation d'une mutéine de β-galactosidase enzymatiquement inactive et immunologiquement active selon l'une des revendications 1 à 3, pour la détermination immunologique de protéines sériques selon

le principe des tests immunoenzymatiques (EIA) à l'aide de l'enzyme $\beta$-galactosidase comme enzyme de marquage pour capturer des anticorps anti $\beta$-galactosidase ou d'autres protéines de liaison dirigées vers la $\beta$-GAL qui peuvent fausser le résultat du test.

**8.** Utilisation selon la revendication 7, caractérisée en ce que la mutéine de $\beta$-galactosidase enzymatiquement inactive est utilisée en même temps qu'une partie Fab d'une immunoglobuline qui n'a pas d'activité de liaison pour l'analyte.

**9.** Utilisation selon la revendication 8, caractérisée en ce que la mutéine de $\beta$-galactosidase enzymatiquement inactive est présente sous forme liée chimiquement à une partie Fab d'une immunoglobuline qui n'a pas d'activité de liaison pour l'analyte.

**10.** Utilisation selon la revendication 8, caractérisée en ce que l'on utilise un mélange constitué par la partie Fab d'une immunoglobuline qui n'a pas d'activité de liaison pour l'analyte et la mutéine de $\beta$-galactosidase enzymatiquement inactive.

**11.** Réactif pour tests immunoenzymatiques (EIA) utilisant la $\beta$-galactosidase comme enzyme de marquage pour éliminer les influences sériques perturbatrices et pour abaisser les valeurs à blanc non spécifiques, caractérisé en ce qu'il contient une mutéine de $\beta$-galactosidase enzymatiquement inactive et immunologiquement active selon la revendication 1, 2 ou 3.

**12.** Réactif selon la revendication 11, caractérisé en ce qu'il contient la mutéine de $\beta$-galactosidase enzymatiquement inactive et immunologiquement active en même temps que la partie Fab d'une immunoglobuline qui n'a pas d'activité de liaison pour l'analyte.

**13.** Réactif selon la revendication 12, caractérisé en ce que la mutéine de $\beta$-galactosidase et la partie Fab d'une immunoglobuline qui n'a pas d'activité de liaison pour l'analyte sont présentes sous forme de mélange.

**14.** Réactif selon la revendication 12, caractérisé en ce qu'il contient, sous forme liée chimiquement, la mutéine de $\beta$-galactosidase et la partie Fab d'une immunoglobuline qui n'a pas d'activité de liaison pour l'analyte.

**Revendications pour les Etats contractants suivants : ES, GR**

**1.** Procédé de préparation par génie génétique d'une protéine mutéine de $\beta$-galactosidase enzymatiquement inactive et immunologiquement active selon la revendication 1, caractérisé en ce que la séquence d'ADN d'un plasmide codant pour la $\beta$-galactosidase est modifiée par mutagenèse dirigée de telle manière qu'après l'expression au moins un acide aminé est remplacé par un autre dans le domaine compris entre les acides aminés 430 et 550 de la protéine et l'activité enzymatique n'est pas supérieure à 1% par rapport à l'enzyme native.

**2.** Procédé selon la revendication 1, caractérisé en ce que l'échange d'acides aminés est opéré au niveau de l'acide aminé Glu 461 et/ou de l'acide aminé Tyr 503.

**3.** Procédé selon la revendication 2, caractérisé en ce que l'acide aminé Glu 461 est remplacé par l'un des acides aminés Ser, Ala, Asp, Tyr et/ou l'acide aminé Tyr 503 est remplacé par l'un des acides aminés Phe, Glu ou Ser.

**4.** Utilisation d'une mutéine de $\beta$-galactosidase enzymatiquement inactive et immunologiquement active préparée selon l'une des revendications 1 à 3, pour la détermination immunologique de protéines sériques selon le principe des tests immunoenzymatiques (EIA) à l'aide de l'enzyme $\beta$-galactosidase comme enzyme de marquage pour capturer des anticorps anti $\beta$-galactosidase ou d'autres protéines de liaison dirigées vers la $\beta$-GAL qui peuvent fausser le résultat du test.

**5.** Utilisation selon la revendication 4, caractérisée en ce que la mutéine de $\beta$-galactosidase enzymatiquement inactive est utilisée en même temps qu'une partie Fab d'une immunoglobuline qui n'a pas d'activité de liaison pour l'analyte.

6. Utilisation selon la revendication 5, caractérisée en ce que la mutéine de β-galactosidase enzymatiquement inactive est présente sous forme liée chimiquement à une partie Fab d'une immunoglobuline qui n'a pas d'activité de liaison pour l'analyte.

7. Utilisation selon la revendication 5, caractérisée en ce que l'on utilise un mélange constitué par la partie Fab d'une immunoglobuline qui n'a pas d'activité de liaison pour l'analyte et la mutéine de β-galactosidase enzymatiquement inactive.

8. Réactif pour tests immunoenzymatiques (EIA) utilisant la β-galactosidase comme enzyme de marquage pour éliminer les influences sériques perturbatrices et pour abaisser les valeurs à blanc non spécifiques, caractérisé en ce qu'il contient une mutéine de β-galactosidase enzymatiquement inactive et immunologiquement active préparée selon la revendication 1, 2 ou 3.

9. Réactif selon la revendication 8, caractérisé en ce qu'il contient la mutéine de β-galactosidase enzymatiquement inactive et immunologiquement active en même temps que la partie Fab d'une immunoglobuline qui n'a pas d'activité de liaison pour l'analyte.

10. Réactif selon la revendication 9, caractérisé en ce que la mutéine de β-galactosidase et la partie Fab d'une immunoglobuline qui n'a pas d'activité de liaison pour l'analyte sont présentes sous forme de mélange.

11. Réactif selon la revendication 9, caractérisé en ce qu'il contient, sous forme liée chimiquement, la mutéine de β-galactosidase et la partie Fab d'une immunoglobuline qui n'a pas d'activité de liaison pour l'analyte.

FIG. 1

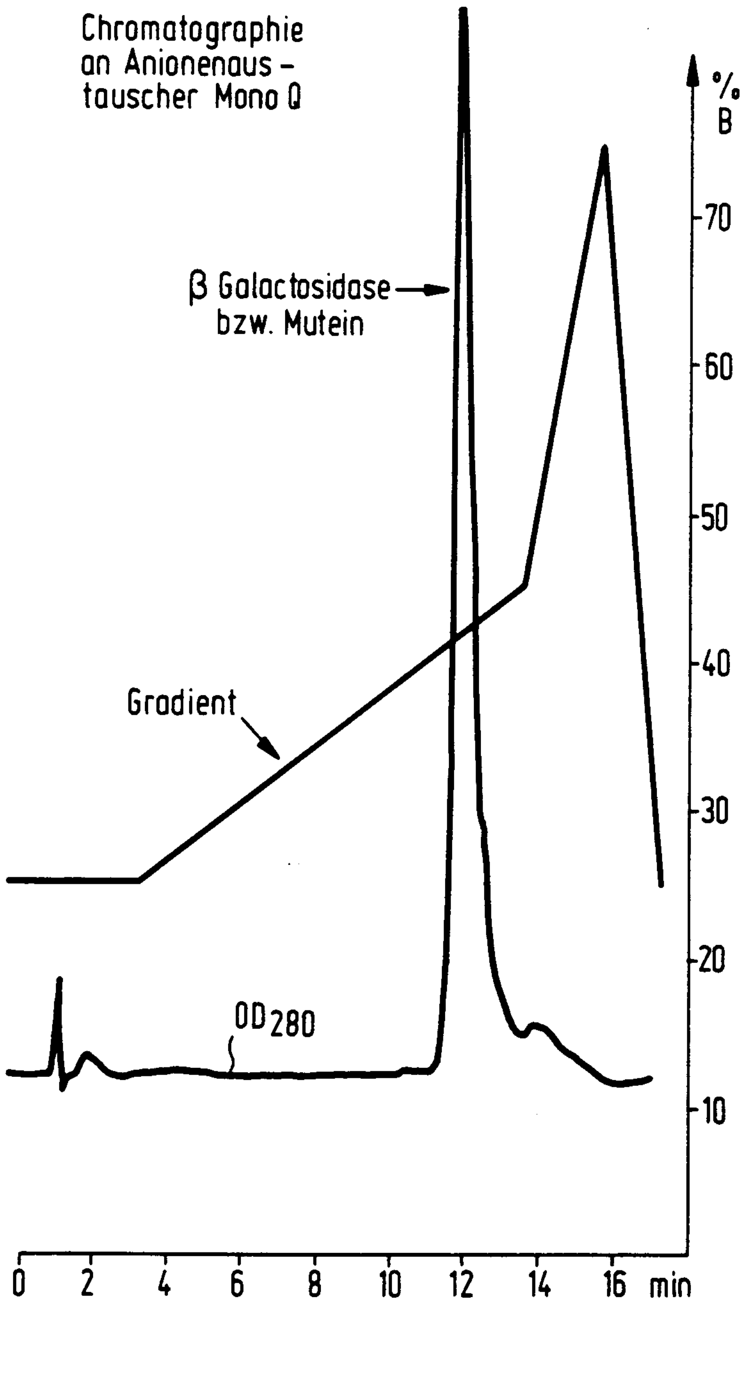
Chromatographie
an Anionenaus-
tauscher Mono Q
β Galactosidase
bzw. Mutein
Gradient
OD280
%
B
70
60
50
40
30
20
10
0
2
4
6
8
10
12
14
16
min

FIG. 2

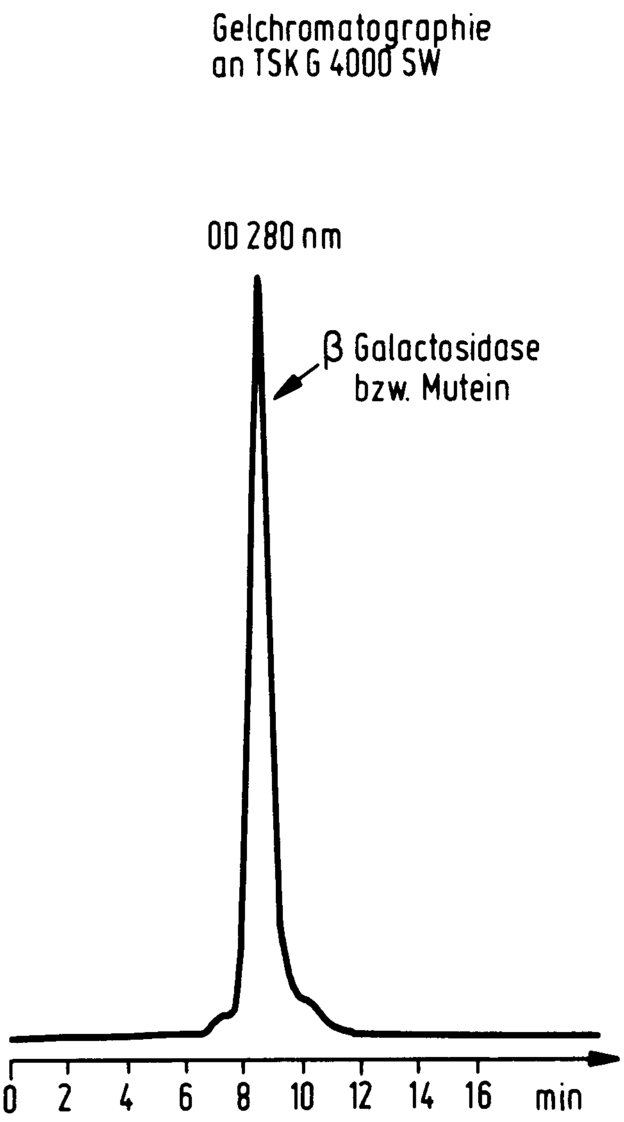
Gelchromatographie
an TSK G 4000 SW
OD 280 nm
β Galactosidase
bzw. Mutein
0
2
4
6
8
10
12
14
16
min

FIG. 3

Polyacrylamid - Gelektrophorese
Gradient 8 - 15 %. ; nativ

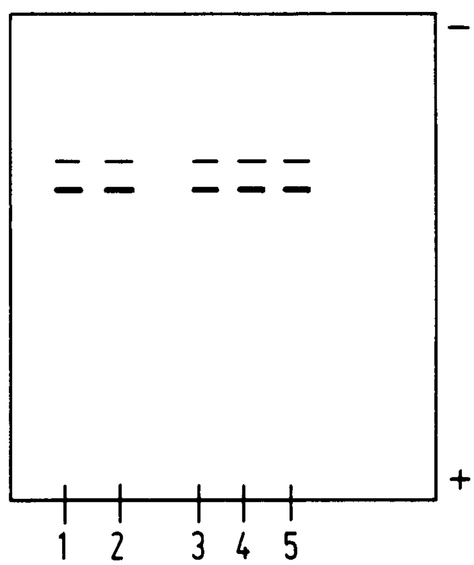


1 Gal Mutein (Ala 461)
2 Gal Mutein (Asp 461)
3 Gal Mutein (Glu 503)
4 native β-Galactosidase
5 Gal Mutein (Ala 503 / Phe 461)

FIG. 4

Vernetzungsprofile der Kupplung von
Maus-Fab mit a.) Gal Mutein (Glu 503)
b.) nativer β-Galactosidase

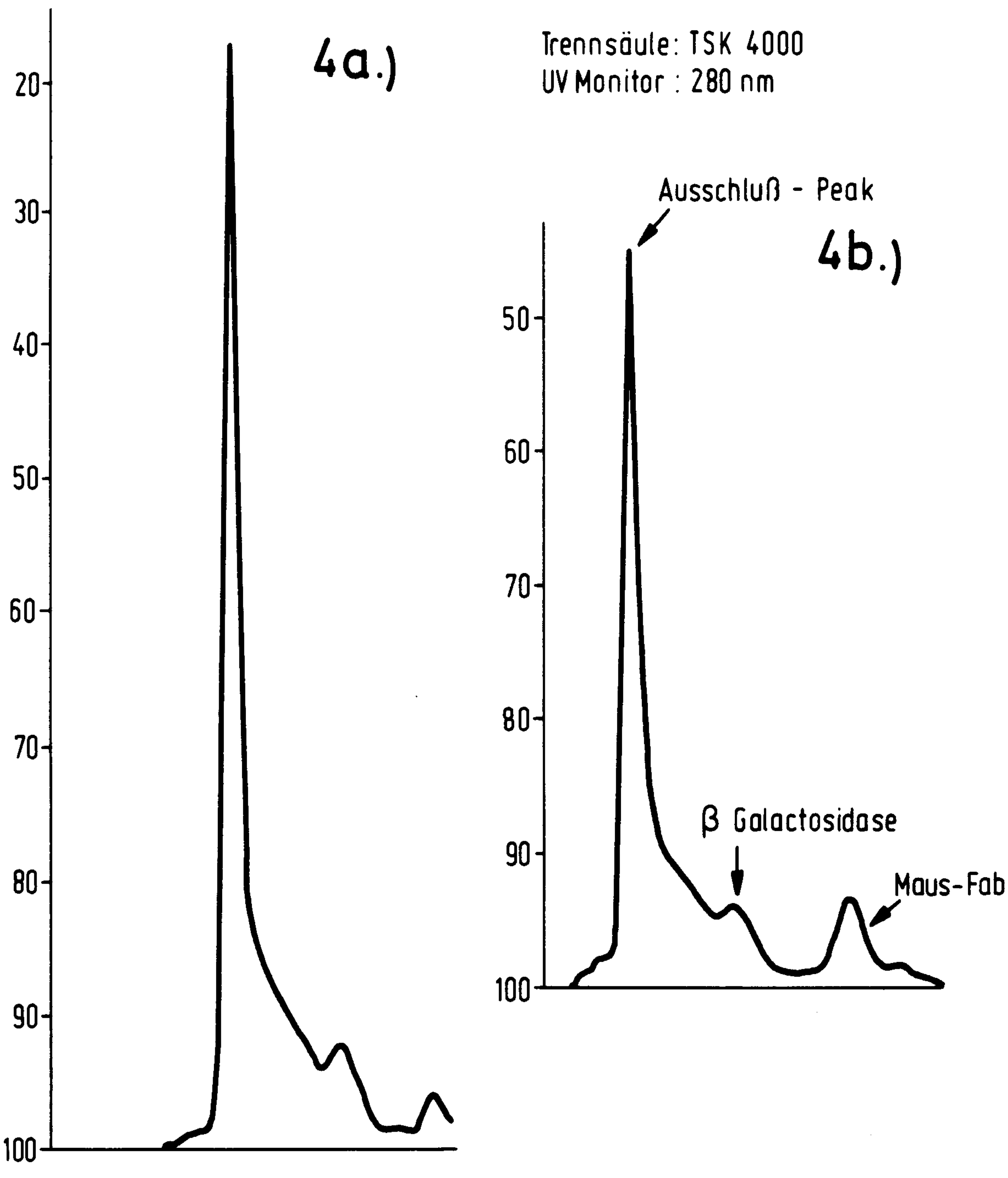

FIG. 5

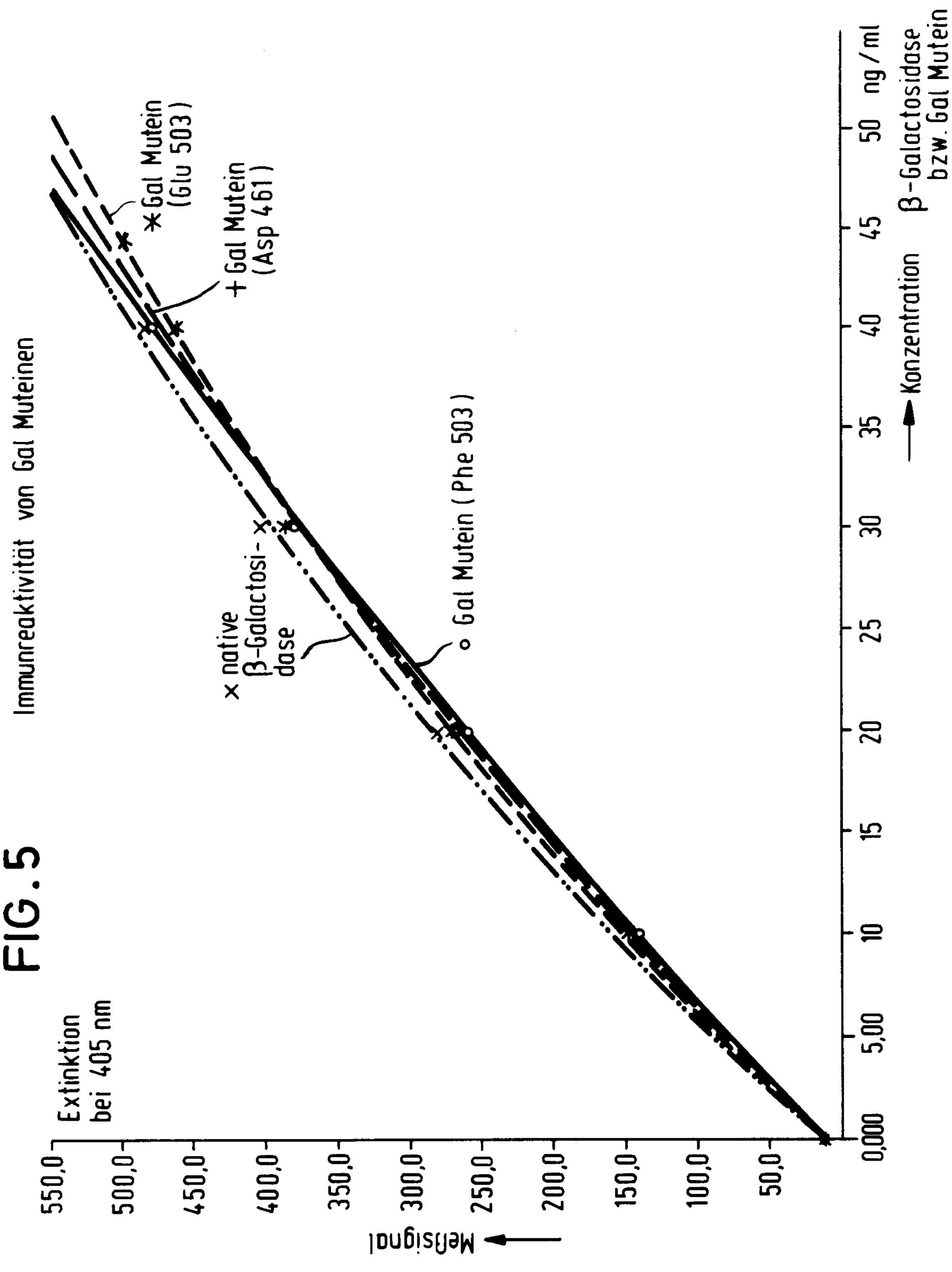
Immunreaktivität von Gal Muteinen
Extinktion bei 405 nm
Meßsignal
550,0
500,0
450,0
400,0
350,0
300,0
250,0
200,0
150,0
100,0
50,0
0,000
5,00
10
15
20
25
30
35
40
45
50 ng/ml
Konzentration
β-Galactosidase bzw. Gal Mutein
× native β-Galactosidase
○ Gal Mutein (Phe 503)
+ Gal Mutein (Asp 461)
✳ Gal Mutein (Glu 503)

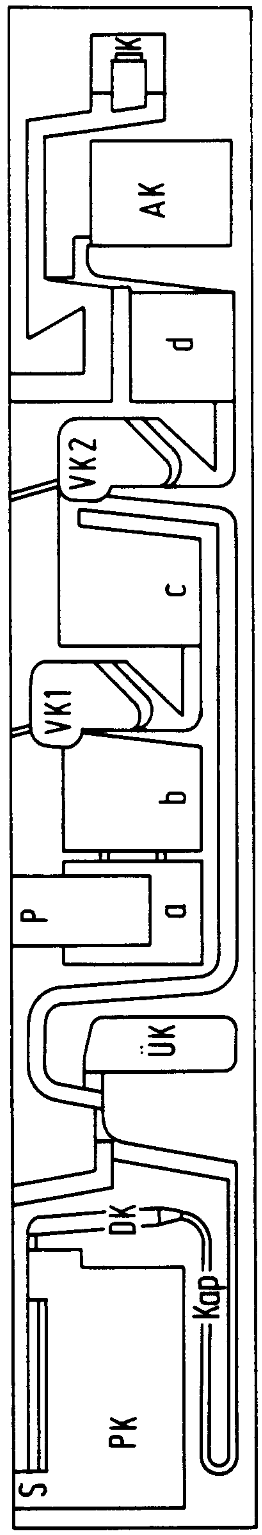


FIG.6